(19)

Europäisches
Patentamt
European
Patent Office
Office européen
des brevets

(11)  **EP 2 908 128 A1**

(12)  **EUROPEAN PATENT APPLICATION**

(43) Date of publication:
**19.08.2015  Bulletin 2015/34**

(51) Int Cl.:
***G01N 33/487*** (2006.01)

(21) Application number: **14155254.7**

(22) Date of filing: **14.02.2014**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Ecole Polytechnique Federale de
Lausanne (EPFL)
1015 Lausanne (CH)**

(72) Inventors:
  • **Radenovic, Aleksandra
    1025 St. Sulpice (CH)**

• **Liu, Ke
  1022 Chavannes-Pres-Renens (CH)**
• **FENG, Jiandong
  1022 CHAVANNES-PRES-RENENS (CH)**
• **Feng, Jiandong
  1022 Chavannes Pres Rennens (CH)**
• **Liu, Ke
  1022 Chavannes Pres Renens (CH)**
• **Radenovic, Aleksandra
  1025 St. Sulpice (CH)**

(74) Representative: **reuteler & cie SA
Chemin de la Vuarpillière 29
1260 Nyon (CH)**

(54)  **Molecular sensing device**

(57)  Molecular sensing system including: a sensing device (5) comprising at least one support layer (10), and an active layer (6) mounted on said support layer and having at least one nano-pore (12) configured for translocation of a molecular analyte (18) therethrough; an electrically conducting liquid (4) in contact with the active layer in a region around said nano-pore; and a signal processing circuit (7) comprising an ionic current circuit (8) configured to generate and measure an ionic current (Ii) in the electrically conducting liquid influenced by the translocation of the molecular analyte through the nano-pore.

Figure 1

**EP 2 908 128 A1**

Printed by Jouve, 75001 PARIS (FR)

**Description**

**Field of the Invention**

The present invention pertains generally to the fields of molecular sensing, in particular solid-state sensing of biomolecules such as DNA , RNA and protein sensing and DNA, RNA sequencing.

**Background of the Invention**

Solid-state nanopores have been developed in order to sense molecular analytes and in particular biomolecules such as DNA, RNA and proteins because they are viewed as being more robust and less dynamic than biopores which are based on protein (toxin) -lipid membrane systems (Clarcke et al., 2009, Nat. Nanotechnol., 4, 265). Solid-state nanopores can operate in various liquid media and pH conditions and their production is scalable and compatible with nanofabrication techniques and can be integrated with other sensing methods that exploit tunneling or local potential gating.

The sensing principle is the same as in bio-engineered pores and ideally, the sequence of nucleotides, genetic information, along a single DNA molecule can be registered by monitoring small changes in the ionic current caused by the transient residing of single nucleotides within a nanometer size pore.

Before sequencing with solid state nanopores, three fundamental requirements on ionic or transverse current signals have to be met: spatial resolution in the order of few nucleotides, high signal to noise ratio and temporal resolution of the signal that allows acquiring enough points per nucleotide using state of the art current-voltage amplifier systems have to be achieved.

However, solid state nanopores exhibit a relatively lower single molecule detection sensitivity compared to biopores due to their intrinsic thickness and lack of control over surface charge distribution: temporal resolution of ionic current in solid state nanopores is on order of 10-50 base pairs/$\mu$s (Branton et al., 2008, Nat. Nanotechnol., 26, 1146). Together with lower ionic current signal to noise ratio, relatively larger sensing region, which is due to the pore membrane thickness, has been major obstacle in achieving sequencing data when using solid state nanopores.

Recently, thin membranes have been proposed to extend the applications of solid-state nanopore to, e.g., detection of short DNA oligomers and differentiation of short nucleotides homopolymers (Venta et al., 2013, ACS Nano, 7, 4629-4636). Several groups have used monolayer graphene (thickness of ~ 0.35 nm) as a nanopore membrane for the detection of DNA translocation (Garaj et al., 2010, Nature, 467, 7312, 190-193) and simultaneous detection of DNA translocation with two synchronized signals, the ionic current in the nanopore and local potential change in the graphene nanoribbon transistor has been reported (Traversi et al., 2013, Nat. Nanotechnol., 8, 939-945).

However, graphene nanopores exhibit strong hydrophobic interactions with DNA that limits their long-term use due to the clogging. Schneider et al. have implemented surface functionalization with pyrene ethylene glycol of graphene nanopores and demonstrated that this process prevents DNA absorption on graphene and renders graphene nanopores usable for extended periods of time (Schneider et al., 2013, Nat. Commun., 4, 2619).

Several attempts have been carried-out for improving temporal resolution of translocating DNA molecules in solid-state nanopores, including using the DNA pore interactions, controlling the electrolyte parameters (temperature, salt concentration, viscosity), nanopore surface functionalization, change in nanopore surface charge though light-control and applying an electrical bias voltage across the nanopore to reduce the mobility of the DNA. *Fologea et al. (*Fologea et al., 2005, Nano Lett, 5, 1734) added glycerol into buffer to increase the viscosity and consequently reduce the mobility of DNA and by controlling the electrolyte temperature, salt concentration, viscosity, electrical bias voltage across the nanopore, obtained a 3 base/$\mu$s, but working with glycerol/water reduced the ionic current signal. In addition, the highest viscosity of the solution that they could use was 5.2 cP.

Therefore, there is a need for selective and sensitive sensing systems for analytes, in particular biomolecular analytes, that allow a rapid analysis at the molecular levels such as for DNA, RNA and protein sequencing.

**Summary of the Invention**

An object of this invention is to provide a system for sensing molecular analytes that enable accurate and reliable detection of the characteristics of analytes at the molecular level.

It is advantageous to provide a system where temporal resolution of the analyte detection is improved.

It is advantageous to provide a system where temporal resolution of the analyte detection is improved such that there is no need of prior manipulation on the analyte to ease detection (e.g. PCR amplification or cutting strands into pieces).

It is advantageous to provide a system for sensing molecular analytes that is economical to produce and to use.

It is advantageous to provide a system for sensing molecular analytes that has a high productivity, in particular that enables rapid detection of the characteristics of analytes at the molecular level.

It is advantageous to provide a system where analyte translocation, in particular DNA translocation through the pore(s) is reduced, while keeping acceptable signal to noise ratios and independently of the analyte length.

Objects of this invention have been achieved by providing a sensing system according to claim 1.

Objects of this invention have been achieved by providing a sensing system according to claim 12.

Disclosed herein, according to a first aspect of the invention, is a molecular sensing system including: a sensing device comprising at least one support layer, and an active layer mounted on said support layer and having at least one nano-pore configured for translocation of a molecular analyte therethrough; an electrically conducting liquid in contact with the active layer in a region around said nano-pore; and a signal processing circuit comprising an ionic current circuit configured to generate an ionic current in the electrically conducting liquid influenced by the translocation of the molecular analyte through the nano-pore, wherein said conducting liquid comprises a first conducting liquid on a first side of the active layer and a second conducting liquid on a second side of the active layer, the first conducting liquid having a viscosity greater than a viscosity of the second conducting liquid.

Disclosed herein, according to a second aspect of the invention, is a molecular sensing system including: a sensing device comprising at least one support layer comprising a support layer orifice, and an active layer mounted on said support layer and having at least one nano-pore configured for translocation of a molecular analyte therethrough, a diameter of the support layer orifice being greater than a diameter of the nano-pore, whereby a portion of the active layer extends over said support layer orifice in a suspended manner; an electrically conducting liquid in contact with the active layer in a region around said nano-pore; and a signal processing circuit comprising an ionic current circuit configured to generate an ionic current in the electrically conducting liquid influenced by the translocation of the molecular analyte through the nano-pore, wherein at least said portion of the active layer extending over said support layer orifice in a suspended manner is of a semi-conducting material, and wherein the a signal processing circuit further comprises a transverse current circuit configured to generate a transverse current in the semi-conducting material.

In an embodiment of the invention, the first conducting liquid comprises a room temperature ionic liquid (RTIL).

In an embodiment of the invention, the room temperature ionic liquid (RTIL) has a viscosity ($cP1$) at room temperature from about 100 centipoises (cP) to about 500 centipoises (cP), for instance from about 100 cP to about 300 cP.

In an embodiment of the invention, the room temperature ionic liquid (RTIL) is selected from a group based on the anion nature: (a) systems based on $AlCl_3$ and organic salts such as 1-butyl-3-methylimidazolium chloride, [bmim] [Cl]; (b) systems based on anions like $[PF_6]^-$, $[BF_4]^-$ and $[SbF_6]^-$; (c) systems based on anions such as $[CF_3SO_3]^-$, $[(CF_3SO_2)_2N]^-$, $[Tf_2N]^-$ and similar; (d) systems based on anions such as alkylsulfates and alkylsulfonates; (e) carboranes ($[CB_{11}H_{12}]^-[CB_{11}H_6Cl_6]^-$, $[CB_{11}H_6Br_6]^-$) and orthoborates.

In an embodiment of the invention, the room temperature ionic liquid (RTIL) comprises hexafluorophosphate anions.

In an embodiment of the invention, the room temperature ionic liquid (RTIL) includes N,N-dialkylimidazolium cations such as dibutyl, dioctyl, dinonyl, didecylimidazolium, 1-Butyl-3-methyl and 1-ethyl-3-methylimidazolium cations ([bmim]+ and [emim] +).

In an embodiment of the invention, the room temperature ionic liquid (RTIL) comprises 1-Butyl-3-methyl and 1-ethyl-3-methylimidazolium cations.

In an embodiment of the invention, the room temperature ionic liquid (RTIL) is preferably 1-butyl-3-methylimi-dazolinom hexafluorophosphate ($BminPF_6$) but in principle RTILs that have high viscosity but have cations that bind preferentially to A,T, G, U or C nucleotides could provide additional benefit in specificity.

In an embodiment of the invention, the second conducting liquid may advantageously comprise or consist in an aqueous liquid comprising an electrolyte.

In an embodiment of the invention, the electrolyte may be potassium chloride (KCl).

In an advantageous embodiment of the invention, the support layer comprises a support layer orifice, a diameter of the support layer orifice being greater than a diameter of the nano-pore, whereby a portion of the active layer extends over said support layer orifice in a suspended manner, and wherein at least said portion of the active layer extending over said support layer orifice in a suspended freestanding manner is of a semi-conducting material.

In an embodiment of the invention, the signal processing circuit further comprises a transverse current circuit configured to generate a transverse current (It) in the semi-conducting material.

In an embodiment the transverse current circuit comprises means to measure the transverse current configured to provide a signal used in the measurement of a characteristic of the molecular analyte as it displaces through the nano pore.

In an embodiment the ionic current circuit comprises means to measure the ionic current configured to provide a signal used in the measurement of a characteristic of the molecular analyte as it displaces through the nano pore.

In an embodiment of the invention, the material of the active layer may advantageously be or comprise $MoS_2$.

In an embodiment of the invention, the material of the semi-conducting material may advantageously be $MoS_2$.

In an embodiment of the invention, the material of the support layer may comprise $SiN_x$.

In an embodiment of the invention, the ionic current circuit may be connected to a pair of Ag/AgCl electrodes coupled to the conducting liquid on opposite sides of the active layer.

The above mentioned features may be combined in any appropriate manner.

An advantageous characteristic of the invention is to provide a system with an atomic thick active layer that can have semi-conducting, conducting or superconductive (depending on the 2D material), having atomically thin membrane improves detection accuracy and reliability. The device can be integrated with transverse detection and this moreover allows providing a plurality of sensing pores in the active layer for measurement of a plurality of molecules in parallel.

An advantageous characteristic of the invention is to provide a system where the detection sensitivity is increased, notably by a decrease of the signal to noise is increased and enhancement of the ionic current signal. According to one aspect, differentiation between nucleotides in solid state pore (hundreds of pico Amperes (pA) in ionic current) is greatly enhanced compared to biological nanopore (tens of pA)

An advantageous characteristic of the invention is to provide a system where pore dimensions may be adjusted to the needs using a highly focused electron beam without the risk of damaging the active layer.

An advantageous characteristic of the invention is to provide a system where the atomically thick active layer is robust enough to avoid being supported by a support layer in the area of the active layer where the pore will be formed, therefore reducing the pore drilling time and improving pore characteristics such as size, shape, and edge properties.

An advantageous characteristic of the invention is to provide a system where the thickness of the nanopore(s) of the active layer is atomically thin.

An advantageous characteristic of the invention is to provide a system having an active layer where the pore diameter can be larger than its thickness.

An advantageous characteristic of the invention is to provide a system where adsorption of the analyte, in particular DNA analytes, onto the active surface is decreased or avoided without the need of additional surface treatments.

An advantageous characteristic of the invention is to provide a system where the viscosity of the conducting fluids can be tuned according to the needs and the analyte to be characterized. The viscosity gradient system exploits room temperature ionic liquid (preferably 1-butyl-3-methylimidazolium hexafluorophosphate (BmimPF6)) as solvent, allowing slowing down DNA translocation speed. This allows adjusting the speed of translocation and thus improving detection accuracy and reliability.

An advantageous characteristic of the invention is to provide a system for sensing molecular analytes that enable accurate and reliable detection of the characteristics of analytes at the molecular level having obtained optimal temporal, spatial resolution while keeping high signal to noise ratio.

In conventional solid state nanopores, analyte translocation speed is too fast to resolve each nucleotide (1-20 nt per $\mu$s). In biological pore, using enzyme to ratchet ssDNA base by base, the resulting speed (1-10 nt per s) is too slow for rapid sequencing. In the present invention one can obtain an optimal speed (10-50 nt per ms) for rapid DNA (1-80 h for whole human genome). The invention advantageously provides a system where temporal resolution of the analyte detection is improved such that there is no need of prior manipulation on the analyte to ease detection (e.g. PCR amplification or cutting strands into individual nucleotides).

An advantageous characteristic of the invention is to provide a system for sensing molecular analytes that is economical to produce and to use by means of nanofabrication and when CVD material is used.

An advantageous characteristic of the invention is to provide a system where analyte translocation, in particular DNA translocation through the pore(s) is reduced, while keeping acceptable signal to noise ratios and independently of the analyte length.

An advantageous characteristic of the invention is to provide a system for sensing molecular analytes that has a high productivity, scalability and integrability in particular that enables rapid detection of the characteristics of analytes at the molecular level with single nucleotide resolution.

Other features and advantages of the invention will be apparent from the claims, detailed description, and figures.

**Brief Description of the drawings**

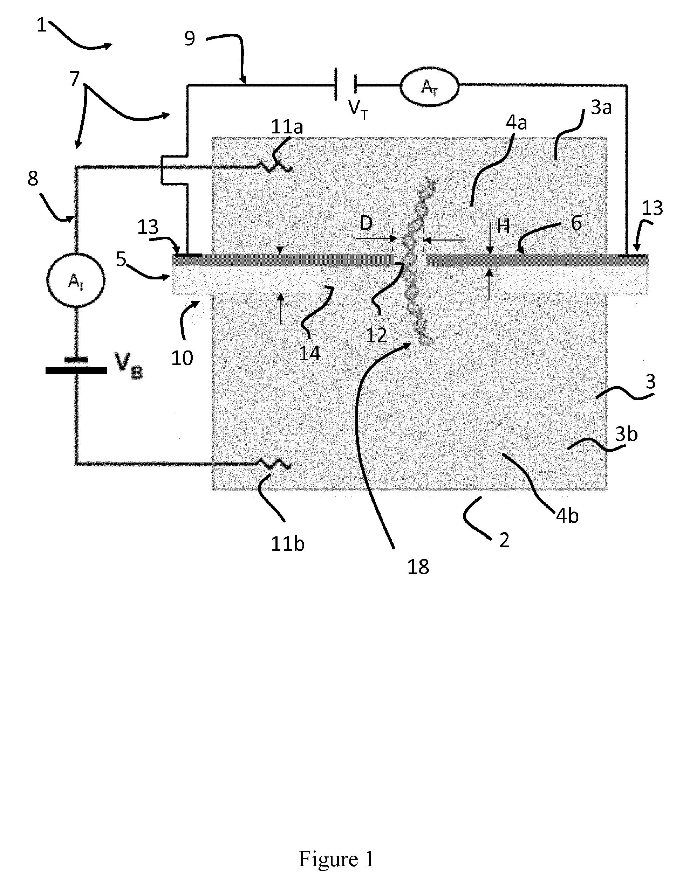

**Figure 1** is a schematic illustration of a molecular sensing system according to the invention.

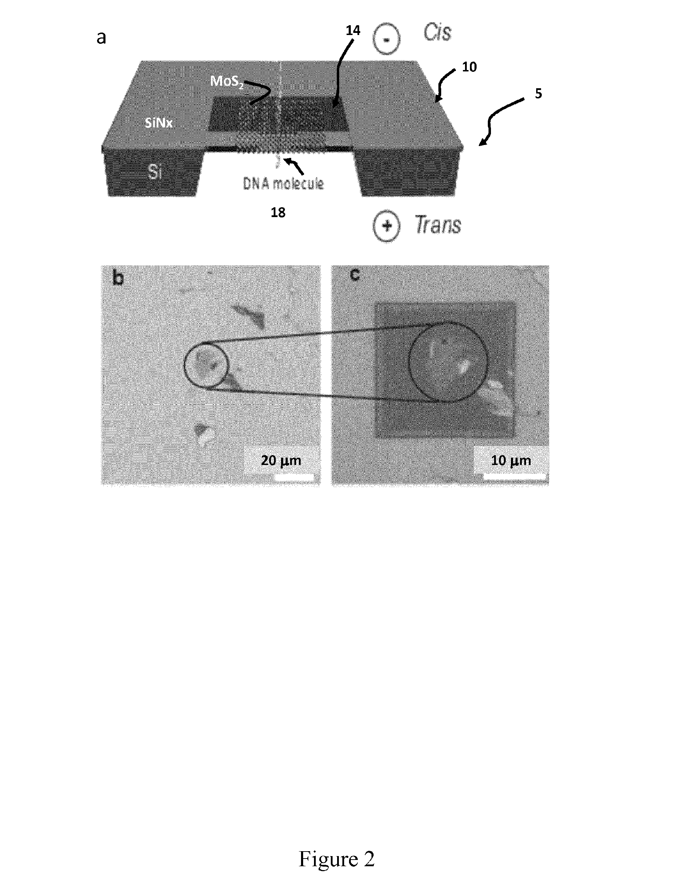

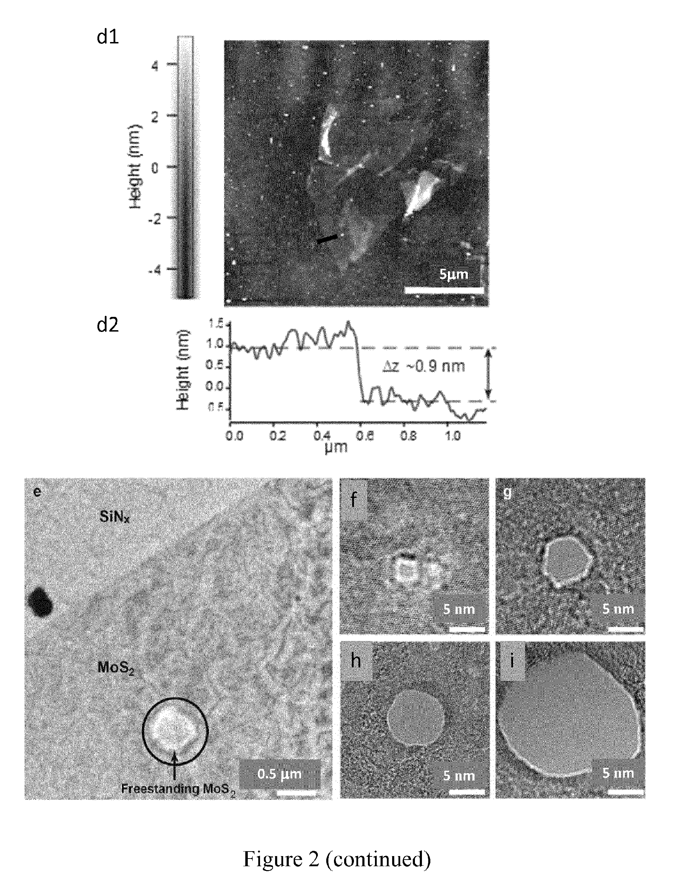

**Figure 2** is an illustration of a molecular sensing system in a configuration as described in Example 1. a) Schematic illustration of an active layer according to the invention made of $MoS_2$ for DNA translocation. Monolayer $MoS_2$ is suspended on a $SiN_x$ support layer that separates two reservoirs containing buffered potassium chloride. Electrical field is applied by a pair of Ag/AgCl electrodes to drive DNA molecules passing through a nanometer size pore on $MoS_2$, while ionic current through the pore is recorded using an Axonpatch low-noise amplifier; b) Optical image of a freshly exfoliated monolayer $MoS_2$ flake (marked with the black circle) exhibiting minimum contrast with respect

to the $SiO_2$ substrate; c) Optical image after the chosen flake has been transferred from the $SiO_2$ substrate to the desired location (a square-shaped opening made by EBL and RIE) on the $SiN_x$ support layer; d1) AFM image of the chosen flake in b) and d2) Height profile is taken from the white line in the image in d1), showing a 9 Å height difference between the surface and the $MoS_2$ flake; e) Low-magnification TEM image of a $MoS_2$ flake fully covering the opening in the $SiN_x$ support layer marked with the black circle. The edge of this flake is clearly seen in the upper part of the image. (f-i) High resolution TEM images of nanopores with various sizes drilled by a focused electron beam. The lattice of $MoS_2$ is visible under such a magnification (1 MX).

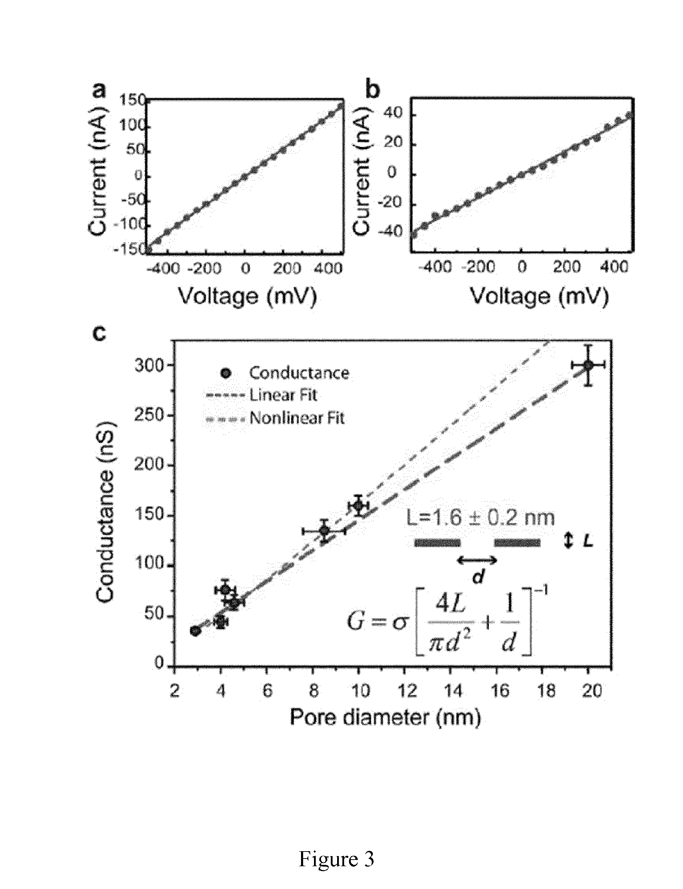

**Figure 3** shows the characterization of a nanopore according to the invention described in Example 1. a) Current voltage characteristic of a 20 nmMoS2 nanopore measured in 2 M KCl; b) Current voltage characteristics of a 5nm $MoS_2$ nanopore measured in 2MKCl; c) Correlation between pore diameters and their conductances. Conductances are derived from linear fits of IV measurements in 2 M KCl with bias voltage swept from -0.5 to +0.5 V. To exclude either leaking pores G > 300 nS or clogged pores G < 10 nS, only devices displaying conductances higher than 10 nS and lower than 300 nS were used. Error bars of the pore diameters indicate the asymmetry of the pores. Inset shows a simple scheme for the thin active layer with a nanopore and related equation to describe conductance, where G is conductance, $\sigma$ is ionic conductivity, L is thickness and d is pore diameter. The nonlinear fitting (small dot line) is based on the equation 1 shown in the inset to subtract L. And the linear fitting (large dot line) is based on the simplified equation 2, $G = \sigma d$, to subtract $\sigma$.

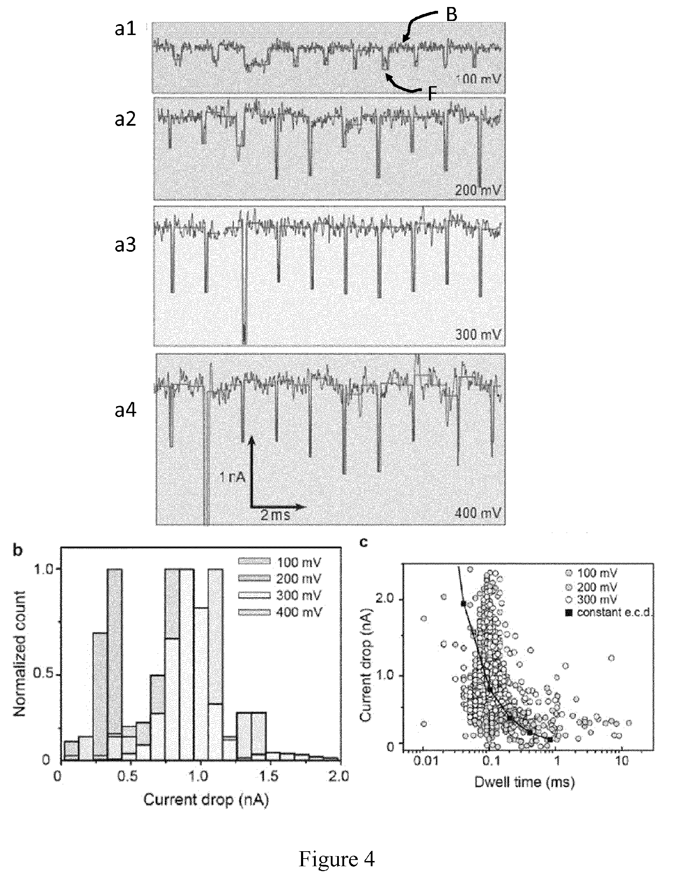

**Figure 4** shows signal analysis as conducted under Example 1. a1- a4) Concatenated events ofpNEB plasmid DNA translocating a 20 nm $MoS_2$ nanopore in 2MKCl. Raw signal (B) and fits (F). Fits are performed using a custom "OpenNanopore" Matlab code; b) Normalized distribution of current amplitude at various voltages; c) Scatter plots of 59, 1823, and 1642 events for 100, 200, and 300 mV, respectively. An electron charge deficit (ECD) method is used to fit the area of individual events.

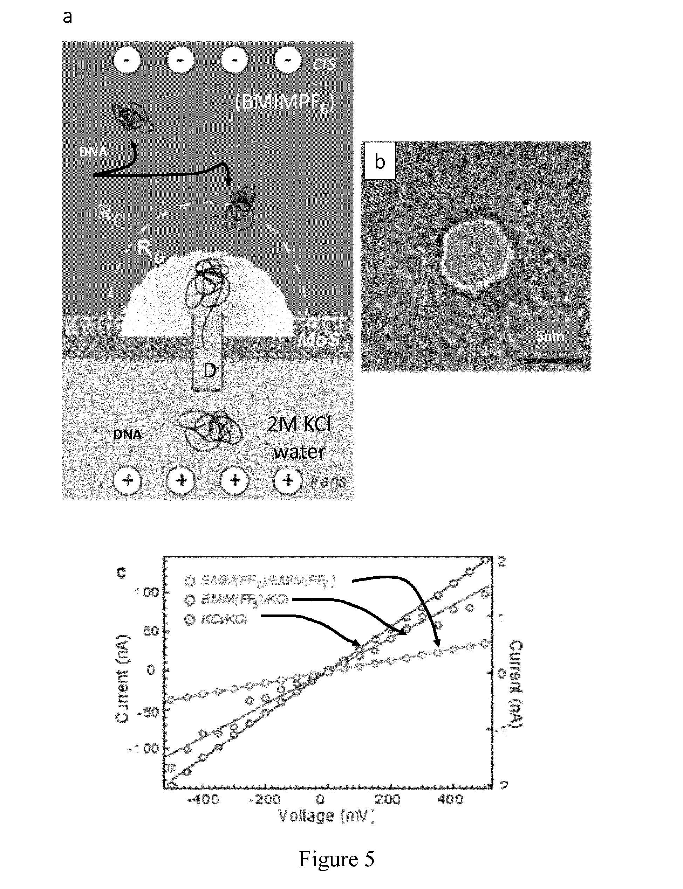

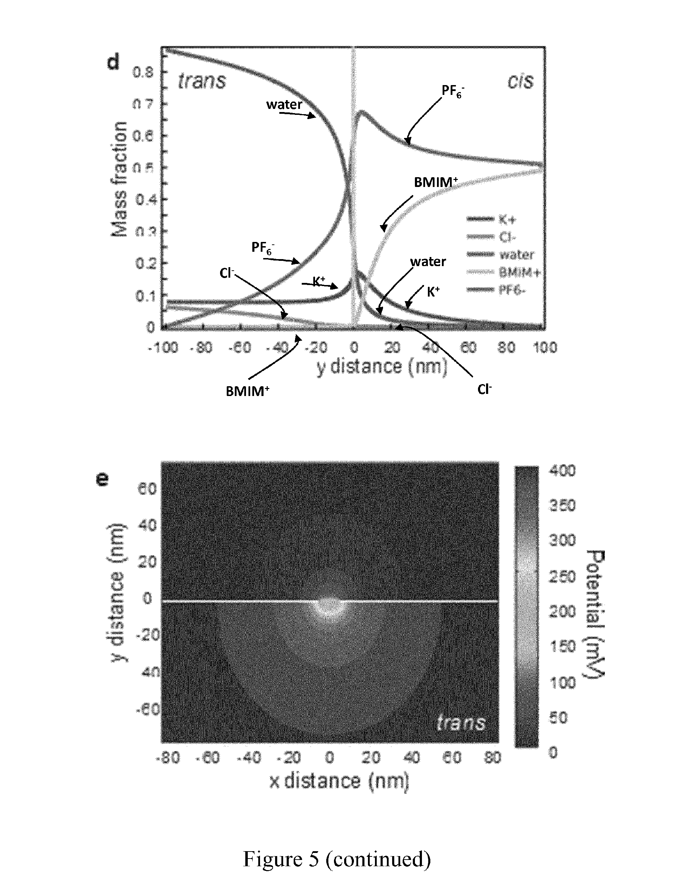

**Figure 5** is schematic and characterization of the RTILs/KCl viscosity gradient system in 2d material nanopore as described in Example 3; a) *Cis* chamber contains RTILs ($BmimPF_6$) while *trans* chamber contains 2M aqueous KCl solution. The two chambers are separated by monolayer $MoS_2$ as active layer with a nanopore. Dynamics of DNA translocation through a monolayer $MoS_2$ nanopore is also displayed. Away from the pore, DNA motion is purely diffusive due to the negligible electric field, but once within the area of capture radius Rc, DNA will be accelerated towards the pore by the force due to electrophoretic and electroosmotic effects, next a part of DNA will undergo conformation change and one end will dive into the pore. The non-translocated part of the DNA polymer -monomers will keep the coil conformation and experience a strong Stokes dragging force from the ionic liquids. Consequently, DNA translocation through the pore can be significantly slowed down; b) Bright-field TEM image of a 5-nm solid-state pore fabricated in a monolayer $MoS_2$ as active layer suspended over a 200 nm x 200 nm etched area formed in the center of 20 $\mu$m big low-stress SiN membrane being 20 nm thick; c) Ohmic current-voltage responses for the $MoS_2$ pore shown in b). IV characteristics are taken at room temperature 2M aqueous KCl solution, in pure $BmimPF_6$ and finally in $BmimPF_6$/2M KCl gradient; d) Mass fraction of water, anions ($PF_6^-$ and $Cl^-$), cations ($Bmim^+$ and $K^+$) as a function of distance from the nanopore; e) Electric potential map evaluated numerically for viscosity gradient system shown in a).

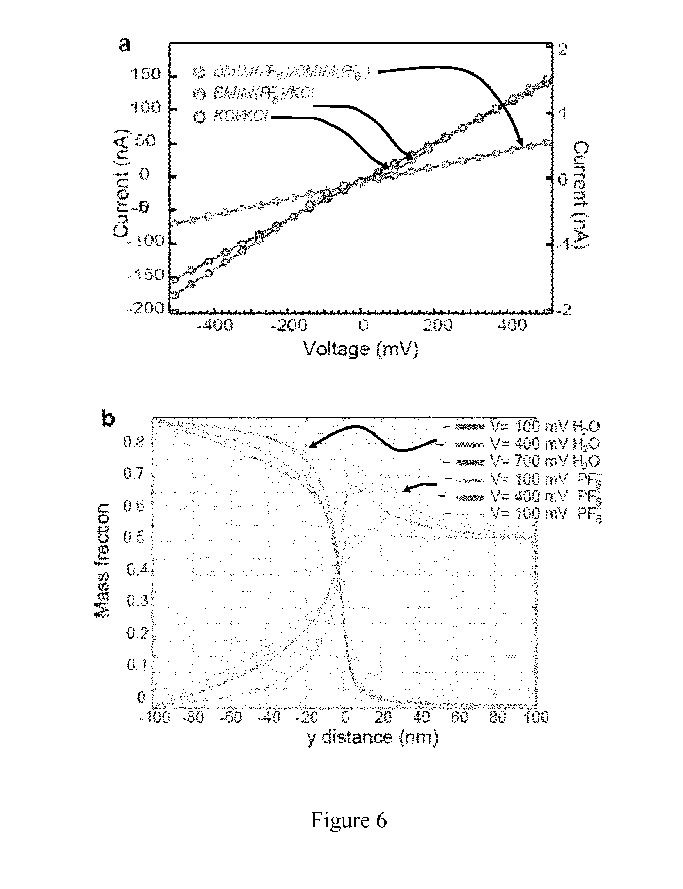

**Figure 6** shows modeling results of the RTILs/KCl viscosity gradient system in 2d material nanopore as described in Example 3; a) COMOSOL modeling of the conductance for the $MoS_2$ pore; b) COMOSOL modeling of the mass fraction of specimen in the vicinity of the $MoS_2$ nanopore.

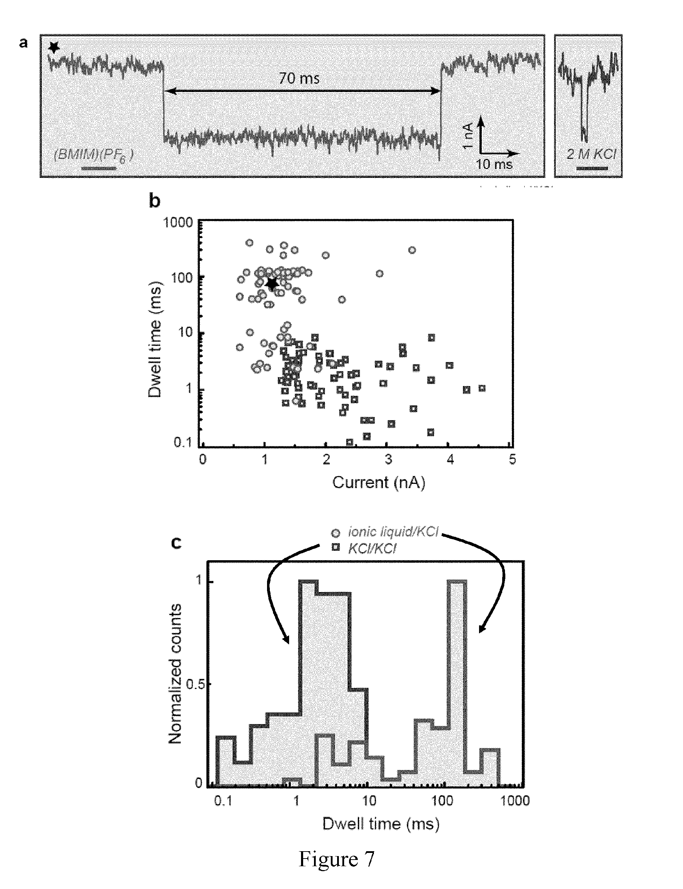

**Figure 7** shows the slowing down DNA translocation by increasing the electroosmotic Stokes force FS in monolayer $MoS_2$ nanopore in a device of Example 3; a) An example of 48.5 kbp $\lambda$-dsDNA translocation event in viscosity gradient system (left). The corresponding current drop represents a single DNA molecule passing through the pore. When compared to the typical translocation trace for 48.5 kbp $\lambda$-dsDNA obtained using a same 20 $MoS_2$ nanopore under no viscosity gradient conditions, DNA the 2 order of magnitude difference in translocation time is evident (right); b) Scatter plots (blockade current versus dwell time) for dwell time versus current signal of $\lambda$-dsDNA trans-location in water (squares) and viscosity gradient system (dots) obtained using a same 20 nm big $MoS_2$ nanopore; c) translocation time histograms corresponding to $\lambda$-dsDNA translocation in water (left), viscosity gradient system (right).

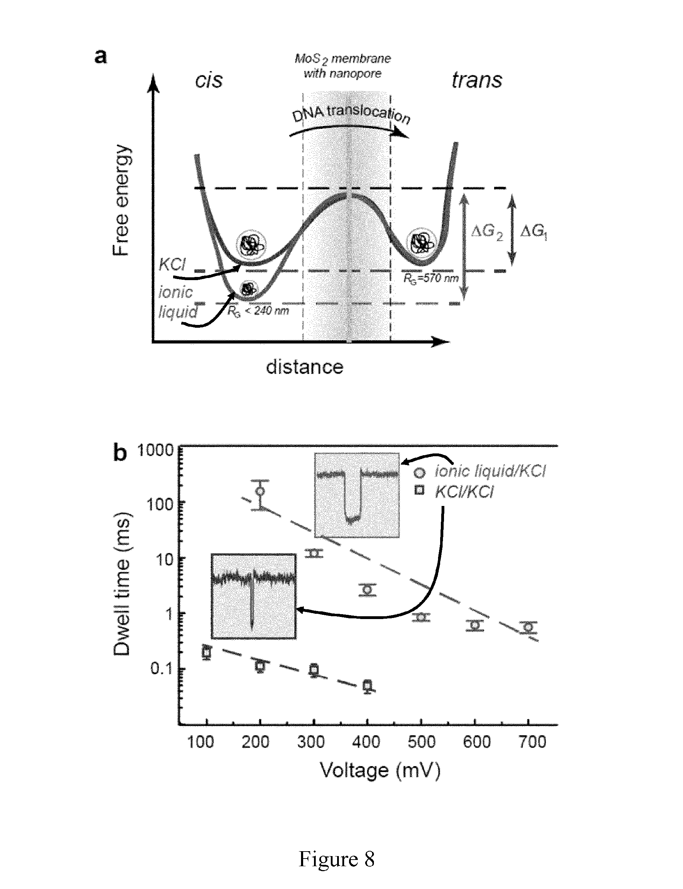

**Figure 8** shows a single molecule DNA translocation through a nanopore probes dynamics of Kramer's theory; a) Schematic representations of single-well free-energy surfaces, for two conditions. The schematics describes intrinsic (i.e. zero voltage) free-energy surface with a well and a barrier to translocation. In the context of the voltage-driven translocation of individual DNA molecules in a nanopore, the well of the free-energy surface corresponds to the random coil DNA configuration in a *cis* chamber with corresponding radius of gyration, while escape over the barrier involves translocation through the nanopore and subsequent adoption of random coil conformation. The free energy should include at least two parts, one from the phase transfer as described using L-J equation, another from entropy part of the DNA coil, but both of these two energy parts give a similar phase as drawn. (the difference is the distance and free energy level); b) Dependence of the translocation dwell time on the applied voltage for pNEB DNA in ionic liquid/ KCl solution (dots) and in KCl/KCl (square). For both conditions exponential dependence reveals that translocation is voltage-activated. Lines are exponential fits to the data.

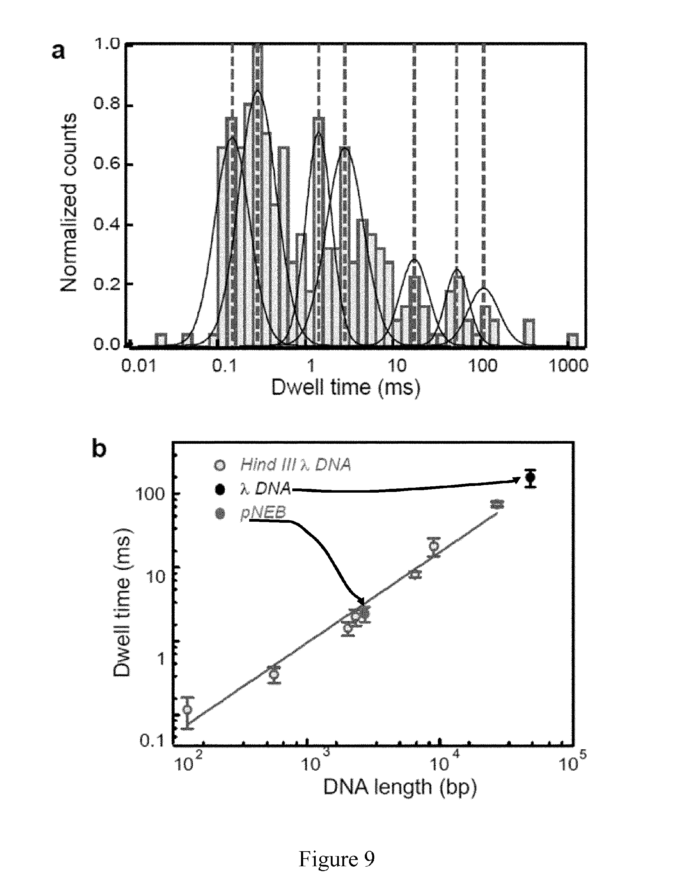

**Figure 9** shows the dependence of the translocation dwell time on the DNA length in a device configuration with a viscosity gradient system as described in Example 3; a) Event distribution of $\lambda$ DNA HindIII digest (564 bp, 2027 bp, 2322 bp, 4361 bp, 6557 bp, 6557 bp, 9416 bp and 23130 bp) translocations in viscosity gradient system; b) Log-log plot of DNA translocation timescales as a function of DNA length (bp) measured using a 20-nm $MoS_2$ pore. Besides $\lambda$ DNA HindIII digest, pNEB and $\lambda$ DNA were added. The line shows the result of a power-law fit to the data, all data points which follow a power law fit exponent of $2v = 1.23$.

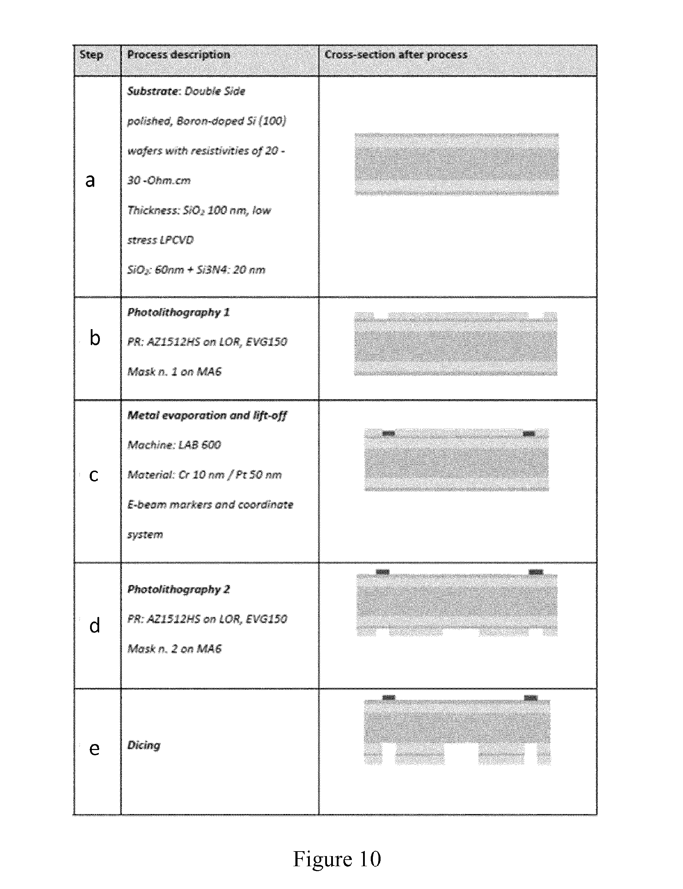

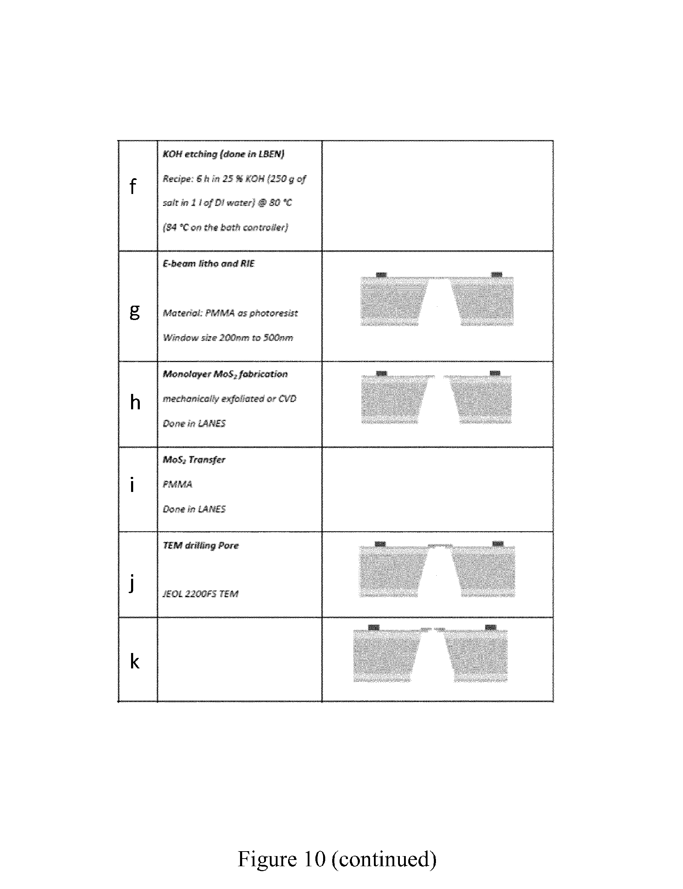

**Figure 10** shows a schematic representation of process steps of preparation of an active layer according to the invention.

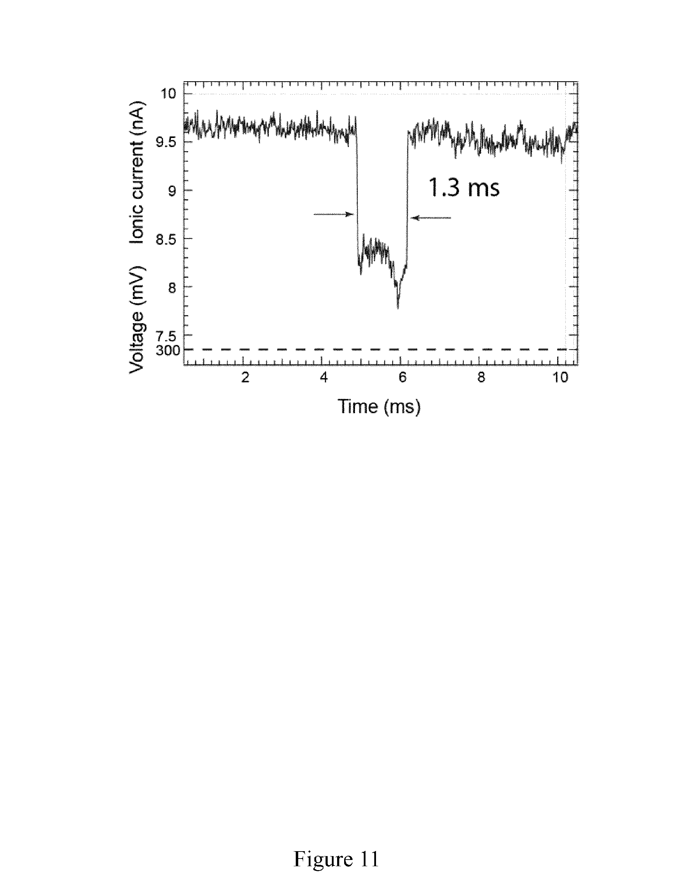

**Figure 11** is a representative translocation event of poly(A)30 in the gradient configuration biased at 300 mV, where cis-chamber is filled with IL and trans-chamber is filled with 2 M KCl. Dry poly(A)30 oligonucleotide is dissolved in IL. A 1.5 nm $MoS_2$ nanopore, calibrated from its ionic conductance, is used. To reduce the noise the device has been cured with PDMS. We observe trace as longer than 1 ms and conductance drops for ssDNA of ~5 nS.

**Detailed Description of the invention**

Referring to the figures, in particular first to Figure 1, a molecular sensing system 1 for sensing a molecular analyte 18 in an electrically conducting liquid 4, comprises a housing 2, a sensing device 5 and a signal processing circuit 7. The housing 2 comprises a first chamber portion 3a containing a first conducting liquid 4a and a second chamber portion 3b containing a second conducting liquid 4b. The sensing device 5 defines the separation between the first chamber portion 3a and a second chamber portion 3b and comprises an active layer 6 and at least one support layer 10 for supporting the active layer in an essentially planar manner. The active layer 6 comprises at least one pore having a pore aperture $Dp$ and a pore height corresponding to the active layer thickness $Hp$. The support layer 10 comprises a support layer orifice 14 having an aperture diameter $Ds$ and an aperture height corresponding to the support thickness $Hs$.

The signal processing circuit 7 comprises an ionic current circuit 8 and a transverse current circuit 9. The ionic current circuit 8 forms an electrical circuit between a voltage source $V_B$ and the electrodes 11a and 11b immerged into the first conducting liquid 4a and the second conducting liquid 4b, respectively and is configured to generate the ionic current $I_i$. The ionic current circuit may further comprise a current measurement circuit portion Ai configured to measure the ionic current $Ii$ to obtain a signal representative of the characteristics of the section of molecular analyte passing through the pore in the active layer. The transverse current circuit 9 forms an electrical circuit between a voltage source $V_T$ electrically connected to the active layer 6 through electrical terminals 13 and generates a transverse current $I_T$ through the active layer. The transverse current circuit may further comprise a current measurement circuit portion At configured to measure the transverse current $It$ to obtain a signal representative of the characteristics of the section of molecular analyte passing through the pore in the active layer.

It is known *per se* that the presence of a translocating molecule locally changes potential and modulates a transverse current as demonstrated in Traversi et al., 2013, Nat. Nanotechnol., 8, 939-945.

According to an aspect of the invention, the active layer 6 is made of a semi-conducting material. The semi-conductive material may advantageously comprise or constitute a layered semi-conductive material which can be exfoliated in thin layer(s), in particular atomically-thin layer(s) (e.g. $MoS_2$ $MoSe_2$, $MoTe_2$, $MoS_2$, $WS_2$, $WSe_2$, $WTe_2$, $TiS_2$, $TiSe_2$, $TiTe_2$, $HfS_2$, $HfSe_2$, $HfTe_2$, $ZrS_2$, $ZrSe_2$, $ZrTe_2$, $NbS_2$, $NbSe_2$, $NbTe_2$, $VS_2$, $VSe_2$, $VTe_2$, $TaS_2$, $TaSe_2$, $TaTe_2$, $TcS_2$, $TcSe_2$, $TcTe_2$, $ReS_2$, $ReSe_2$, $ReTe_2$, $PdS_2$, $PdSe_2$, $PdTe_2$, $PtS_2$, $PtSe_2$ and$PtTe_2$). According to an advantageous embodiment, the active layer comprises $MoS_2$ thin layers or is a $MoS_2$ monolayer.

In an aspect of the invention, the provision of a freestanding suspended portion of semiconductor active layer through which the nano pore is formed allows to benefit from the advantage of being able to apply a transverse current in the active layer and measure the signal affected by the translocation of the molecular analyte, as well as being able to form an accurate nano pore in a very thin active layer without having to drill through a relatively thick support layer because of the suspended freestanding portion of active layer.

Thin layers of $MoS_2$ with good quality suitable for use in a device according to the invention can be prepared by both exfoliation and chemical vapor deposition (CVD) *(Novoselov et al., PNAS, 2005, 102, 10541-1053; Liu et al. 202, Nano Lett., 12, 1538-1544).

According to another aspect, the active layer is from about 0.3 nm to about 1 nm thick.

According to another aspect, pores in the active layer are nanometer sized, typically from about 1 nm to 20 nm diameter and from about 0.3 nm to 1 nm thickness

According to another aspect, the pores are drilled into the active layer with a highly focused electron beam (e.g. in a transmission electron microscope).

According to another aspect, the support layer can be a $SiN_x$ glass, or quartz (or any other material that provides low capacitance membrane) with a support orifice according to the invention. According to a further aspect, the support orifice has typically a diameter of from about 200 nm to about 500 nm and from about 20 nm to 50 nm thick.

According to a further aspect, the support layer can be coated with some curing layer such as polydimethylsiloxane (PDMS), while leaving the $MoS_2$ nanopore exposed in order to reduce the dielectric noise. Alternatively, support layer can be a quartz, glass, or any other material that provides low capacitance membrane based support.

According to another aspect, a viscosity gradient is created between the first electrically conducting liquid in the first chamber portion (*cis* chamber) and the second electrically conducting liquid in the second chamber portion (*trans* chamber). In particular, according to a further aspect, the first electrically conducting liquid is a "room temperature ionic liquid" (also known as "RTIL") and the second electrically conducting liquid is an aqueous ionic solution (e.g. water and KCl or any inorganic salts such as LiCl, NaCl, $MgCl_2$ $CaCl_2$ etc). "Ionic liquids" are non-aqueous electrolytes only composed of loosely coordinated bulky ions forming pairs of organic cations and anions and have been largely described (Chiappe et al., 2005, J. Phys. Org. Chem, 18: 275-297; Keskin et al., 2007, J. of Supercritical Fluids, 43, 150-180; Carda-Broch, 2003, Anal. Bioanal. Chem., 375: 191-199).

According to a further aspect, RTILs according to the invention have a viscosity at room temperature not less than about 100 cP. According to a further aspect, RTILs according to the invention have a viscosity at room temperature from about 100 cP to about 10'000 cP, in particular from about 100 cP to about 500 cP and more particularly from about 100 cP to about 300 cP, tuneable in a wide range upon RTILs chemical composition. According to a further aspect, RTILs according to the invention may be selected from the following group based on the anion nature: (a) systems based on $AlCl_3$ and organic salts such as 1-butyl-3-methylimidazolium chloride, [bmim][Cl]; (b) systems based on anions like $[PF_6]^-$, $[BF_4]^-$ and $[SbF_6]^-$; (c) systems based on anions such as $[CF_3SO_3]^-$, $[(CF_3SO_2)_2N]^- \equiv [Tf_2N]^-$ and similar; (d) systems based on anions such as alkylsulfates and alkylsulfonates; (e) carboranes ($[CB_{11}H_{12}]^-$, $[CB_{11}H_6Cl_6]^-$, $[CB_{11}H_6Br_6]^-$) and orthoborates. According to a particular embodiment, a RTIL according to the invention comprises hexafluorophosphate anions. According to a further aspect, RTILs according to the invention may include N,N-dialkylimidazolium cations such as dibutyl, dioctyl, dinonyl, didecylimidazolium, 1-Butyl-3-methyl and 1-ethyl-3-methylimidazolium cations ($[bmim]^+$ and $[emim]^+$). According to a particular embodiment, a RTIL according to the invention comprises 1-Butyl-3-methyl and 1-ethyl-3-methylimidazolium cations. According to a further aspect, a RTIL according to the invention is 1-butyl-3-methylimidazolium hexafluorophosphate ($BminPF_6$). According to one aspect, $BmimPF_6$ has the advantage of presenting a broad viscosity window of 10-400 cP which is tunable according to needs by changing chemical composition of RTILs and it is a friendly solvent for bio-molecules, while, most importantly, it exhibits good ionic conductivity 1.4 mS cm$^{-1}$. In contrast, low conductivity of glycerol limited previous attempts to narrow viscosity window (1.2-5 cP) and consequently achieved only modest improvement in DNA translocation time 3 base/$\mu$s.

According to one embodiment is provided an example of a device according to the invention as schematized in Figure 2a, where few layers or even monolayer $MoS_2$ (active layer) are suspended on the pre-etched square-shaped opening on the 20 nm thick supporting $SiN_x$ membranes (support layers). In this configuration, the analyte (e.g. DNA) can translocate through subnanometer thick $MoS_2$ active layer instead of 20 nm thick $SiN_x$ to achieve a better spatial resolution. A transfer method (Petrone et al., 2012, Nano Lett., 12, 2751-2756) was used to suspend monolayer and few-layer $MoS_2$ on SiNx supporting layers and high resolution electron microscopy technique was used to sculpt nanopores in variable diameters on the in free-standing $MoS_2$ active layers. Pore forming example is provided in the Example section below. Micromechanical exfoliation method (Nanoselov, 2004, Science, 306, 666-669) was used to exfoliate few-layer $MoS_2$ from natural $MoS_2$ bulky material onto the surface of substrates covered with 270 nm $SiO_2$ chips with fiducial markers. An optical microscope is used to identify single and few layers $MoS_2$ by their contrast under illumination.

As shown in Figure 2b, monolayer $MoS_2$ shows minimum contrast with respect to the substrate. The coordinates of chosen flakes were recorded and used for the further transfer onto the $SiN_x$ support layer. To verify the thickness of this chosen flake, atomic force microscopy (AFM) was used to obtain its height profile, as shown in Figure 2d. The thickness is 9 Å from AFM measurements and is indicative of a monolayer, which is consistent with the optical observation. Subsequently, this flake was transferred from the silicon dioxide substrate to a square-shaped opening (ranging from 200 to 500 nm in size to reduce electric noise when flake in contact with ionic buffer) on the target $SiN_x$ supporting layer using a standard graphene transfer method (Petrone et al., 2012, Nano Lett., 12, 2751-2756). Figure 1c is the optical image after a successful transfer of the flake shown in Figure 2b,d to the desired location (marked by the black circle) on the support layer. TEM with low magnification was used to search for the chosen flake. Figure 2e illustrates the full coverage of the opening in $SiN_x$ by the flake (marked by the black circle), preventing ionic current leakage. The $MoS_2$ lattice can be clearly resolved in the high-magnification image with the diffraction pattern (DP) reflecting the hexagonal symmetry of $MoS_2$. The drilling process of the pore lasts only for several seconds after which a nanometer-sized pore appears within the $MoS_2$ layer. This is another indication that the pore was drilled through only few atoms of $MoS_2$. Therefore, a good thermal and mechanical stability is highly preferred for such a short drilling period, especially in the case of small pores. The beam is preferably blanked for several minutes before the drilling process starts in order to minimize the drift for the both beam and the active layer sample. Figure If-i shows several examples of nanopores with various sizes.

Moreover, in some cases the number of layers can be identified by inspecting the folded edges of the flake. The current-voltage (IV) characteristics of $MoS_2$ nanopores with various sizes (2-20 nm) was investigated when immersed in the 2 M KCl buffered solution as electrically conducting liquid. Figure 3 a,b shows examples of IV curves measured in the KCl buffer, exhibiting linear and symmetric characteristics from 500 to 500 mV. According to the model first proposed by Wanunu et al., 2010, Nat. Nanotechnol., 5, 807-814 and later Kowalcyzk et al., 2011, Nanotechnology, 22, 315101 the conductance of nanopore can be described by equation 1:

$$G = \sigma \left[ \frac{4L}{\pi d^2} + \frac{1}{d} \right]^{-1}$$

$$(1)$$

where $\sigma$, L and d are the ionic conductivity of 2 M KCl (20 S m$^{-1}$), membrane thickness and nanopore diameter, respectively. Two major elements associated with pore geometry contribute to the conductance, namely channel resistance (the first term in the equation) and access resistance (the second term in the equation). Figure 3c shows a plot of all working devices in this study. Using a nonlinear fit, L was obtained with a value of 1.6 (0.2 nm, reflecting an atomically thin feature of the $MoS_2$ active layers. For an ultrathin membrane, channel resistance is much smaller than the access resistance. Therefore, the conductance can also be expressed as equation 2:

$$G = \sigma d$$

As a result, a nearly linear relationship between pore conductance and pore diameter is expected. From a linear fitting, $\sigma$ was found with a value of 17.5 $\pm$ 1.5 S m$^{-1}$, which is in a good agreement with ionic conductivity of 2 M KCl (20 S m$^{-1}$). Therefore, both fits are suitable. At any given pore size, the conductance from $MoS_2$ nanopore is much larger than that of $SiN_x$. Moreover, no influence of the number of layers (always less than 4) was observed on the conductance since the pore diameter is larger than its thickness.

Ideally, (K$^+$, Cl$^-$) ions flow in two directions under the influence of the electrical field (ionic current circuit) through a nanopore resulting in a constant ionic current, namely, the baseline current. DNA translocation will give rise to temporary blockades in ionic pore current manifested by a decrease in ionic current on the time-scale of approximately 100 $\mu$s to 10 ms, as shown in Figure 4a. pNEB plasmid DNA was first translocated through a 20 nm diameter $MoS_2$ nanopore to eliminate the multiple conformation issue. Two parameters, the amplitude of blockage and dwell time are used to quantify individual translocation events. Cumulative sums (CUSUM) algorithm was used to detect events automatically and extract above-mentioned parameters for each event (Raillon et al., 2012, Nanoscale, 4, 4916-4924). Events are concatenated with short segments of the baseline signal preceding and following them. Due to the circular shape of the pNEB plasmid, all events have only one level indicating a single conformation. The signal amplitude also increases upon raising the applied voltage as shown in Figure 4b. Mean signal amplitudes are 0.3, 0.7, 0.9, and 1.1 nA for 100, 200, 300, and 400mV, respectively. The ratio $2*(2.2/d)^2$ was used to calculate theoretical blockage, where d = 20 nm in this case. Blockage percentage is 2.4%, in accordance with the experimental value of 1.5%. Scatter plots are used to describe the statistics of DNA translocation as shown in Figure 4c. For voltages above 200mV, fast translocation is observed with a most probable dwell time of <100 $\mu$s. But for 100 mV, a much broader distribution (200 $\mu$s full-width at half-maximum) is observed with a mean dwell time of <300 $\mu$s.

A constant electron charge deficit (ECD) to fit the scatter plot (Figure 4c) for various Voltages was used. As a result, a value of <500 ke is obtained, which is at least five times of previously reported data for 3 kbp DNA *(Fologea et al., 2005, supra)*. This increase is mainly due to the increased blockage amplitude because of the greatly improved sensitivity of $MoS_2$ nanopores compared to conventional $SiN_x$ nanopores (current drop here found of <400 pA at 100 mV as compared to <100 pA at 100 mV, earlier reported).

It is very advantageous to have a good signal-to-noise ratio (SNR), preferably more than 6, for event detection. In this configuration SNR was > 10 (100 pA RMS noise and <nA signal). The percentage of device failure (conductance higher than 300 nS due to leakage or lower than 10 nS due to pore clogging) in solution is surprisingly low (<30%).

When pNEB DNA was translocated through a 5 nm diameter $MoS_2$ pore according to this configuration under the same experimental conditions (representative concatenated traces observed), both mean current amplitude and mean dwell time are larger for the 5 nm pore compared to the 20 nm pore as observed on a scatter plot, implying a local interaction between the edge of the MoS$_2$ pore and the DNA molecule. To extend this statement, this interaction happens only when DNA is sliding through the edge of pore with the effect of retarding DNA translocation. For larger pores (20 nm), translocations tend to be in a frictionless manner.

The present example of a device of the invention shows that the active layers with a single size-tunable nanopore can be produced with good yield and very low device failure rate when working in high ionic strength buffers and that translocation of various types of DNA trough MoS$_2$ pores exhibits a signal amplitude that is five times higher than in the case of solid-state Si$_3$N$_4$ active layers and a SNR of more than 10. These features are highly desirable for event detection which were used to show the electric-field induced unfolding of a 48 kbp long DNA molecule within the nanopore which manifests itself in the quantization of the current drop.

Therefore, semi-conducting active layers, such as MoS$_2$ nanopore active layers can compete with graphene nanopore active layers in terms of spatial resolution and possibly better performance for transverse detection of analyte translocation.

According to another embodiment is provided an example of a device according to the invention as schematized in Figure 5a where a 5 nm MoS$_2$ nanopore is used in combination with a viscosity gradient between the first conducting liquid and the second conducting liquid, the first conducting liquid being pure BmimPF$_6$ where a conductivity of 200 nS was achieved. (Figure 5d). Fig. 5d shows mass fraction of water molecules, anions and cations as a function of distance from the nanopore at 400 mV transmembrane bias (V$_B$). Sub-nanometer membrane thickness ensured that via diffusion process relatively high number of water molecules diffused from *trans* (second chamber portion) into *cis* chamber (first chamber portion), similarly, anions and cation molecules diffused into respective chamber. Modeled conductances shown in Fig. 6a are in good agreement with the measurements. Interestingly, mass fraction of water molecules in *cis* chamber weakly depends on transmembrane bias, while PF$_6$ anions diffusion is strongly affected Fig. 6b. This supports that a well-characterized viscosity gradient system has been built. Translocation of a 48.5 kbp λ-dsDNA was achieved by adding the plasmid into the *cis* chamber filled with pure BmimPF$_6$. In order to minimize nanopore-DNA interaction that can also significantly contribute to the DNA translocation retardation, slightly bigger MoS$_2$ nanopores (~20 nm) were used. Fig. 7a displays the typical current trace of translocating lambda DNA molecule in viscosity gradient system at 400 mV, if compared to the typical current trace in 2M aqueous KCl solution obtained using a same pore and applying the same voltage, one can observe temporal improvement and no reduction in the current amplitude drop. The amplitudes of ionic current blockage and the translocation times, are extracted using custom Matlab routines. The average translocation time is 130ms for lambda DNA in ionic liquids, while 1.4 ms in KCl solution. At this point, for the same pore, same DNA molecule and the same biasing electric field, a retardation factor can be introduced and can be defined as equation 3:

$$r = \frac{v_{H_2O}}{v_{Bmim(PF_6)}} = \frac{\varepsilon_{H_2O}\, \eta_{Bmim(PF_6)} \left( \zeta_{DNA}^{H_2O} - \zeta_{W}^{H_2O} \right)}{\varepsilon_{Bmim(PF_6)}\eta_{H_2O} \left( \zeta_{DNA}^{Bmim(PF_6)} - \zeta_{W}^{Bmim(PF_6)} \right)}$$

$$(3)$$

where ε is the dielectric constant of the electrolyte, η the viscosity of the electrolyte, ζDNA and ζw the zeta potentials on DNA and pore surfaces respectively in corresponding electrolytes. In the absence of electroosmotic flow this expression simplifies, and by measuring the ζDNA in 2M aqueous KCl solution and in BmimPF$_6$ using phase analysis light scattering (Zetasizer Nano instrument Malvern Ltd., U.K.) retardation factor of 2120 is obtained and is predominantly due to the increase in the viscosity and decrease in the relative dielectric constant in the viscosity gradient system.

In general, single molecule DNA translocation process can be viewed as voltage driven over the barrier. In Figure 8a, free-energy surface is schematized with a well and a barrier to translocation for viscosity gradient system and for the 2M KCl aqueous solution. In viscosity gradient system, λ-DNA adopts random coil configuration with gyration radius 240 nm, while in 2M KCl aqueous solution corresponding gyration radius is 570 nm. From the schematics, for applied voltage smaller than the free energy barrier associated with the translocation process, one can expect low probability of translocation since they are only diffusion driven. On the other hand, increasing the applied voltage reduces the effective barrier, and therefore significantly increases the probability of translocation. For the same pore, when working in the 2M KCl aqueous solution translocations start to be observed at much lower voltage 100 mV compared to viscosity gradient system (200 mV) as shown in Figure 8b. This observation led to schematize higher energy barrier for the viscosity gradient system. Figure 8b details the comparison between translocation times of pNEB DNA for a wide range of applied voltages and two different systems (2M KCl aqueous solution and viscosity gradient system). DNA

translation in both systems obeys a power-law scaling:

$$\tau \sim V^{\alpha}$$

(4)

where $\tau$ is dwell time while V is the ionic voltage applied to the electrodes 11a and 11b (Figure 1). For both conditions, exponential dependence was observed that reveals that translocation is voltage-activated. Lines are exponential fits to the data. Translocation times of $\lambda$ DNA HindIII digest (564 bp, 2027 bp, 2322 bp,4361 bp, 6557 bp, 6557 bp, 9416 bp and 23130 bp) was measured as a function of their length. Next, translocation experiments were conducted with $\lambda$DNA and plasmid pNEB. From Figure 9a, it is possible to identify respective dwell time for each fragment. In contrast to previous reports, where temporal dynamics has been DNA length dependent (Wananu et al., 2008, Biophys. J., 95, 4716), temporal dynamics in the present system is independent on the DNA length, even slightly stronger for the longer DNA molecules, which is an ideal condition for DNA sequencing. Fig. 9b shows a clear power-law scaling of the dwell-time with the contour length of the DNA fragment while a least-squares fit to the data yields v=1.23 Flory exponent consistent to the theoretical prediction and previous experimental results obtained in water (v=1.26-1.27) (Storm et al., 2005, Nano Lett., 5, 1193). Finally, short oligonucleotides poly (dA) 30 (Figure 11), poly (dT) 30, poly (dG) 30 and poly (dC) 30 were translocated through pores smaller than 3 nm.

Altogether, these results support that DNA translocation can be regulated to meeting single base time resolution through the use of device of the invention where a viscosity gradient is created between the first chamber portion and the second chamber portion. Such a viscosity gradient system can be not only used in standard ionic sensing experiment (ionic circuit only) but it can be combined with other kind of nanopore sensing such as transverse current signal detection. The use of the ultrahigh viscosity of ionic liquids in the first chamber portion allows controlling DNA translocation at room temperature.

According to one aspect, a system according the invention may be useful for example for DNA, RNA sequencing and detection of DNA methylation, low weight biomolecule detection, eg micro RNA, si RNA and detection of any low molecular weight biomarkers for early diagnosis (e.g. cancer etc.).

The invention having been described, the following examples are presented by way of illustration, and not limitation.

## Examples

### Example 1: Preparation of a sensing device of the invention

The 20 nm thick supporting $SiN_x$ membranes are manufactured in a standard procedure using anisotropic KOH etching. Membrane sizes vary from 10 to 50 $\mu$m depending on the size of the backside opening. Electron beam lithography (EBL) and reactive ion etching (RIE) is used to make a square-shaped opening with a size of 200-500 nm on the membrane. $MoS_2$ flakes are first mechanically exfoliated onto substrates with 270 nm $SiO_2$ and fiducial markers. Optical microscope (Olympus IX51) was used to identify few layers or even monolayer flakes by their optical contrast. The thickness of chosen flakes is further confirmed by AFM measurements (Asylum Research Cypher). The method of transferring flakes to the square-shaped opening located on the $SiN_x$ membrane is similar to the widely used graphene transfer method. Electron beam drilling is performed in a JEOL 2200FS TEM operated at an acceleration voltage of 200 kV. Before loading in the microscope, the samples are annealed at 400 °C under a $H_2$/Ar flux in order to remove any residual organic material left on the surface from the microfabrication processing and prevent hydrocarbon deposition. The fabrication process is detailed in Example 2. Membranes are imaged in the TEM mode with low magnification (<10 kx) in order to identify the location of suspended $MoS_2$ flake. Drilling is performed by focusing the beam with the condensor lens aperture (CLA) at high magnification (600 kxto 1 Mx). The nanopore membrane chip is mounted inside custom flow cell as soon as possible after drilling, otherwise stored in a desiccator with controlled humidity. After mounting the sample in the microfluidic setup, the wetting of the pore is facilitated by flushing the microfluidic system with a water-ethanol (v/v, 1:1) solution. It is crucial to inspect and remove bubbles trapped in the microfluidic channels. An Axopatch 200B patch clamp amplifier (Molecular Devices, Inc., Sunnyvale, CA) is used to record the ionic current in the single cell configuration with a sampling rate of 100 kHz and lowpass filter of 10 kHz. A NI PXI-4461 card was used for data digitalization and custom-made LabView software for data acquisition. Chlorinated Ag/AgCl electrodes are inserted in both cis and trans reservoirs and connected to the Axopatch 200B. DNA samples (pNEB193, plasmid 2.7 kbp, New England; $\lambda$-DNA, 48 kbp, New England) are buffered with filtered and degassed 2 M KCl, 10 mM Tris, 1 mM EDTA and pH 7.4 and adjusted to a final concentration of 1-10 ng/$\mu$L. Finally, the solution containing DNA is injected into the *cis* chamber of the flow cell, which is grounded using the Ag/AgCl electrode. Each type of DNA is translocated in at least two different devices and representative and reproducible results and analysis are presented. Data analysis is performed offline using a custom open source Matlab code, named Open- Nanopore (http://lben.epfl.ch/page-79460-en.html), for event detection. Results are presented under Figures 2 to 4.

**Example 2: Active layer preparation**

Samples were prepared starting from boron-doped 380-$\mu$m-thick silicon chips (Ø100 mm) with resistivities of 20 - 30 Ohm*cm. Chips were coated on both sides with a 60-nm-thick layer of $SiO_2$ and a 20 nm top layer of low-stress $SiN_x$ (Figure 10 a-c). The thickness of the $SiN_x$ was chosen for structural reasons, and the thickness of the $SiO_2$ was chosen to optimize the visibility of the 2D materials, enhancing the optical contrast with the bare. A square window (~500 $\mu$m $\times$ 500 $\mu$m Figure 10d-e) was opened in the $SiO_2$/$SiN_x$ layer on the back side by EBL and RIE. Chips were then wet etched in KOH to remove the silicon and the front side $SiO_2$ layer, resulting in a square $SiN_x$ membrane (~20 $\mu$m $\times$ 20 $\mu$m (Figure 10f). Before depositing 2D material on the chip, the substrate was pre-patterned using EBL, opening of 200-500 nm $\times$ 200-500 nm square in a methyl methacrylate (MMA)/PMMA electron-beam resist double layer. The square is located in the center of the nitride membrane. A subsequent RIE process was used to create small opening in the membrane, as shown in (Figure 10g) 2D membranes can be formed either using exfoliated single layer flakes or CVD grown material. **Exfoliated 2D** material flakes (preferably from dichalgonenides, or graphene) are first mechanically exfoliated onto substrates with 270 nm $SiO_2$ and fiducial markers (Figure 10h). Next we use optical microscope (Olympus IX51) to identify few layers or even monolayer flakes by their optical contrast. The thickness of chosen flakes is further confirmed by AFM measurements (Asylum Research Cypher) Figure 2.. The method of transferring flakes to the square-shaped opening located on the $SiN_x$ membrane is similar to the widely used graphene transfer method (Figure 10i).
**Graphene CVD** Large-area graphene films were grown on copper foils. The growth took place under the flow of a methane/argon/hydrogen reaction gas mixture at a temperature of 1,000 °C. At the end of the growth, the temperature was decreased rapidly and the gas flow turned off. The copper foils were then coated with poly(methylmethacrylate) (PMMA) and the copper etched away, resulting in a centimetre-scale graphene film ready to be transferred onto the chips with membranes. This graphene was single layer, continuous and had good electronic properties.

**Molybdenum disulfide CVD**

Single layer (SL) $MoS_2$ have been grown by chemical vapour deposition (CVD) on sapphire c-plane (Figure 10h). Substrates were cleaned by sonication in acetone, isopropanol and DI-water, consecutively, followed by 1h etching in $H_2SO_4$:$H_2O_2$ (3:1). They were then loaded into a 2-inch CVD furnace and placed face-down under over a crucible containing ~5 mg $MoO_3$ ($\geq$99.998% Alfa Aesar) with another crucible containing ~350 mg of sulfur ($\geq$99.99% Aldrich) located upstream. The CVD growth is performed at atmospheric pressure with flowing ultrahigh-purity argon. The growth recipe is following: set 300°C with 200 s.c.c.m. for 10 min, ramp to 700°C at 50°C min$^{-1}$ with 10 s.c.c.m., set 700°C for 10 min, cool to 570°C with 10 s.c.c.m., open furnace and flow 200 s.c.c.m. for rapid cooling.
Electron beam drilling is performed in a JEOL 2200FS TEM operated at an acceleration voltage of 200 kV. Before loading in the microscope, the samples are annealed at 400 °C under a $H_2$ / Ar flux in order to remove any residual organic material left on the surface from the microfabrication processing and prevent hydrocarbon deposition. Membranes are imaged in the TEM mode with low magnification (< 10 kX) in order to identify the location of suspended $MoS_2$ flake. Drilling is performed by focusing the beam with the condensor lens aperture (CLA) at high magnification (600 kX - 1 MX) resulting in pores that can have diameters from 1 nm -20 nm (Figure 10j-k).

**Example 3: Molecular sensing device with viscosity gradient**

Exfoliated or CVD grown $MoS_2$ thin layers were transferred either from $SiO_2$ or sapphire substrates and suspended on the SiNx membranes, and nanopores were further drilled using a JEOL 2200FS high resolution transmission electron microscope (HRTEM) as described in *Liu et al. (in press).* The chips with nanopore were sealed by silicone o-rings between two polymethylmethacrylate (PMMA) chamber as reservoirs. After mounting, the whole flow cell was flushed with H2O:ethanol (v:v, 1:1) solution and wetted for at least 30 mins. Then 2 M KCl solution buffered with 10 mM Tris-HCl and 1mM EDTA at pH 7.0 and Bmin$^+$PF6$^-$ (Aldrich-Sigma) were injected to perform current-voltage (IV) characteristics measurements. A pair of chlorinated Ag/AgCl electrodes immersed in two reservoirs and connected to a Axopatch 200B patch clamp amplifier (Molecular Devices, Inc. Sunnyvale, CA) that was used to measure ionic current as a function of time, namely, Ii(t).For detection of the homopolymers of short nucleotides, and when working with the small pores <5nm we employed Chimera preamplifier (Chimera Instruments, New York, NY, USA). The device was running at the applied voltage for at least 1 hr to perform blank experiments. DNA samples were diluted to pure Bmin$^+$PF$_6^-$

by mixing 10 $\lambda$ L DNA stock solution with Bmin$^+$PF6$^-$. DNA samples (pNEB193, plasmid 2.7 k bp, New England; $\lambda$-DNA, 48 k bp, New England) were purchased from commercial supplier, aliquoted and stored at -20 °C before the use. NI PXI-4461 card was used for data digitalization and a custom-made LabView 13 software for data acquisition. The sampling rate is 100k Hz and a built-in low pass filter is used. Data analysis is performed offline using a custom open source Matlab code, named OpenNanopore (*Raillon et al., 2012, supra*) (http://lben.epfl.ch/page-79460-en.html), for event detection. Each type of DNA were translocated in at least two different devices, and representative and reproducible results and analysis are presented.

*COMSOL Modeling*

Numerical solutions were performed using the COMSOL 4.2 Multiphysics finite element solver in 3D geometry, imposing a cylindrical symmetry along the axis of the nanopore. The full set of Poisson-Nerst-Planck (PNP) equations were solved, with the boundary conditions at the $MoS_2$ corresponding to idealized, uncharged membrane impermeable to ions. Simulated $MoS_2$ conductances in 2M aqueous KCl solution, viscosity gradient system and pure RT ionic liquid (Bmin$^+$PF$_6$) was found to differ by <5% from the measured values presented in Figure 2c. A DNA molecule was modeled as a 50 nm long stiff insulating rod threading the nanopore along its axis.

### *List of elements referenced (figures 1 and 2)*

**1** molecular sensing system

**2** housing

**3** sensing chamber

**3a** first chamber portion (*cis* chamber portion)
**3b** second chamber portion (*trans* chamber portion)

**5** sensing device

**6** active layer

$\rightarrow$ semi-conducting
**12** pore(s) (nano)

**Dp** pore diameter
**Hp** active layer thickness

**10** support layer(s)

**14** support layer orifice

**Ds** support layer orifice diameter
**Hs** support layer thickness

**15** support layer end section

**7** signal processing circuit

**8** ionic current circuit

**V$_B$** voltage source
**A$_i$** ionic current measuring system

**I$_i$** ionic current

**11a, 11b** electrodes

**9** transverse current circuit

**V$_T$** voltage source
**At** transverse current measuring system

**It** transverse current

**13** electrical terminals

Connection interface to computing and user interface system

**4** electrically conducting liquid

**4a** first conducting liquid

$\rightarrow$ room temperature ionic liquid (RTIL)

**4b** second conducting liquid

$\rightarrow$ aqueous liquid with electrolyte (KCl) (ionic buffer)

**18** molecular analyte

**Claims**

1. **Molecular sensing system** including:

   - a sensing device (5) comprising at least one support layer (10), and an active layer (6) mounted on said support layer and having at least one nano-pore (12) configured for translocation of a molecular analyte (18) therethrough;
   - an electrically conducting liquid (4) in contact with the active layer in a region around said nano-pore; and
   - a signal processing circuit (7) comprising an ionic current circuit (8) configured to generate an ionic current *(Ii)* in the electrically conducting liquid influenced by the translocation of the molecular analyte through the nano-pore,
   wherein said conducting liquid comprises a first conducting liquid (4a) on a first side of the active layer and a second conducting liquid (4b) on a second side of the active layer, the first conducting liquid having a viscosity (cP1) greater than a viscosity (cP2) of the second conducting liquid.

2. Molecular sensing system according to the preceding claim, wherein the first conducting liquid comprises a room temperature ionic liquid (RTIL).

3. Molecular sensing system according to the preceding claim, wherein the room temperature ionic liquid (RTIL) has a viscosity (cP1) at room temperature from about 100 cP to about 500 cP.

4. Molecular sensing system according to claim 2 or 3, wherein the room temperature ionic liquid (RTIL) is selected from a group based on the anion nature: (a) systems based on $AlCl_3$ and organic salts such as 1-butyl-3-methylimidazolium chloride, [bmim][Cl]; (b) systems based on anions like $[PF_6]^-$, $[BF_4]^-$ and $[SbF_6]^-$; (c) systems based on anions such as $[CF_3SO_3]^-$, $[(CF_3SO_2)_2N]^-$, $[Tf_2N]^-$; (d) systems based on anions such as alkylsulfates and alkylsul-fonates; (e) carboranes ($[CB_{11}H_{12}]^-$, $[CB_{11}H_6Cl_6]^-$, $[CB_{11}H_6Br_6]^-$) and orthoborates.

5. Molecular sensing system according to claim 2 or 3, wherein the room temperature ionic liquid (RTIL) comprises hexafluorophosphate anions.

6. Molecular sensing system according to claim 2 or 3, wherein the room temperature ionic liquid (RTIL) includes N,N-dialkylimidazolium cations.

7. Molecular sensing system according to claim 2 or 3, wherein the room temperature ionic liquid (RTIL) is 1-butyl-3-methylimidazolium hexafluorophosphate ($BminPF_6$).

8. Molecular sensing system according to any of the preceding claims, wherein the second conducting liquid is an aqueous liquid comprising an electrolyte.

9. Molecular sensing system according to the preceding claim, wherein the electrolyte is potassium chloride (KCl).

10. Molecular sensing system according to any preceding claim, wherein the support layer (10) comprises a support layer orifice (14), a diameter ($Ds$) of the support layer orifice being greater than a diameter ($Dp$) of the nano-pore, whereby a portion of the active layer extends over said support layer orifice in a suspended manner, and wherein at least said portion of the active layer extending over said support layer orifice in a suspended manner is of a semi-conducting material.

11. Molecular sensing system according to the preceding claim, wherein the signal processing circuit (7) further comprises a transverse current circuit (9) configured to generate a transverse current (It) in the semi-conducting material.

12. **Molecular sensing system** including:

   - a sensing device (5) comprising at least one support layer (10) comprising a support layer orifice (14), and an active layer (6) mounted on said support layer and having at least one nano-pore (12) configured for translocation of a molecular analyte (18) therethrough, a diameter ($Ds$) of the support layer orifice being greater than a diameter ($Dp$) of the nano-pore, whereby a portion of the active layer extends over said support layer orifice in a suspended manner;
   - an electrically conducting liquid (4) in contact with the active layer in a region around said nano-pore; and
   - a signal processing circuit (7) comprising an ionic current circuit (8) configured to generate and measure an ionic current ($Ii$) in the electrically conducting liquid influenced by the translocation of the molecular analyte through the nano-pore,
   wherein at least said portion of the active layer extending over said support layer orifice in a suspended manner is of a semi-conducting material, and wherein the a signal processing circuit (7) further comprises a transverse current circuit (9) configured to generate a transverse current (It) in the semi-conducting material.

13. Molecular sensing system according to any preceding claim, wherein the material of the active layer comprises $MoS_2$.

14. Molecular sensing system according to any preceding claim, wherein the material of the support layer comprises $SiN_x$.

15. Molecular sensing system according to any preceding claim, wherein the ionic current circuit (8) is connected to a pair of Ag/AgCl electrodes coupled to the conducting liquid on opposite sides of the active layer.

Figure 1

Figure 2

Figure 2 (continued)

Figure 3

Figure 4

Figure 5

Figure 5 (continued)

Figure 6

Figure 7

Figure 8

Figure 9

| Step | Process description | Cross-section after process |
|------|---------------------|----------------------------|
| a | *Substrate: Double Side polished, Boron-doped Si (100) wafers with resistivities of 20 - 30 -Ohm.cm*<br><br>*Thickness: SiO₂ 100 nm, low stress LPCVD*<br><br>*SiO₂: 60nm + Si3N4: 20 nm* | |
| b | *Photolithography 1*<br><br>*PR: AZ1512HS on LOR, EVG150*<br><br>*Mask n. 1 on MA6* | |
| c | *Metal evaporation and lift-off*<br><br>*Machine: LAB 600*<br><br>*Material: Cr 10 nm / Pt 50 nm*<br><br>*E-beam markers and coordinate system* | |
| d | *Photolithography 2*<br><br>*PR: AZ1512HS on LOR, EVG150*<br><br>*Mask n. 2 on MA6* | |
| e | *Dicing* | |

Figure 10

| | | |
|---|---|---|
| f | *KOH etching (done in LBEN)*<br><br>*Recipe: 6 h in 25 % KOH (250 g of*<br>*salt in 1 l of DI water) @ 80 °C*<br>*(84 °C on the bath controller)* | |
| g | *E-beam litho and RIE*<br><br>*Material: PMMA as photoresist*<br>*Window size 200nm to 500nm* | |
| h | *Monolayer MoS₂ fabrication*<br>*mechanically exfoliated or CVD*<br>*Done in LANES* | |
| i | *MoS₂ Transfer*<br>*PMMA*<br>*Done in LANES* | |
| j | *TEM drilling Pore*<br><br>*JEOL 2200FS TEM* | |
| k | | |

Figure 10 (continued)

Figure 11

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

Application Number

EP 14 15 5254

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2013/021815 A1 (HITACHI HIGH TECH CORP [JP]; OHURA TAKESHI [JP]) 14 February 2013 (2013-02-14) * paragraph [0041] - paragraphs [0047], [0077], [0078], [0118] - [0122]; figures 1,2,4,5 * | 1-11, 13-15 | INV. G01N33/487 |
| X | US 2014/021047 A1 (SHIM JEO-YOUNG [KR] ET AL) 23 January 2014 (2014-01-23) * paragraphs [0008], [0028] - [0036]; figures 1,5 * | 1-11, 13-15 | |
| X | F. TRAVERSI ET AL: "Detecting the translocation of DNA through a nanopore using graphene nanoribbons", NATURE NANOTECHNOLOGY, vol. 8, no. 12, 17 November 2013 (2013-11-17), pages 939-945, XP055134764, ISSN: 1748-3387, DOI: 10.1038/nnano.2013.240 * the whole document * | 1-11, 13-15 | |

-----

-----

-----

-/--

TECHNICAL FIELDS
SEARCHED (IPC)

G01N
C12Q

### INCOMPLETE SEARCH

The Search Division considers that the present application, or one or more of its claims, does/do
not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| The Hague | 18 August 2014 | Gilow, Christoph |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04E07)

Europäisches
Patentamt

European
Patent Office

Office européen
des brevets

# PARTIAL EUROPEAN SEARCH REPORT

Application Number

EP 14 15 5254

## DOCUMENTS CONSIDERED TO BE RELEVANT

| Category | Citation of document with indication, where appropriate, of relevant passages | Relevant to claim |
|---|---|---|
| X | WO 2012/073009 A2 (IMP INNOVATIONS LTD [GB]; ALBRECHT TIM [GB]; IVANOV ALEKSANDAR PETROV) 7 June 2012 (2012-06-07) * abstract; figure 1a * ----- | 1-11, 13-15 |
| A | WO 2011/130312 A1 (ELECTRONIC BIOSCIENCES LLC [US]; ERVIN ERIC N [US]) 20 October 2011 (2011-10-20) * page 15, line 11 - line 19; figure 2 * ----- | 1-11, 13-15 |
| A | MENI WANUNU ET AL: "Electrostatic focusing of unlabelled DNA into nanoscale pores using a salt gradient", NATURE NANOTECHNOLOGY, vol. 5, no. 2, 20 December 2009 (2009-12-20), pages 160-165, XP055134839, ISSN: 1748-3387, DOI: 10.1038/nnano.2009.379 * the whole document * ----- | 1-11, 13-15 |

CLASSIFICATION OF THE
APPLICATION (IPC)

TECHNICAL FIELDS
SEARCHED        (IPC)

EPO FORM 1503 03.82 (P04C10)

1

Europäisches
Patentamt

European
Patent Office

Office européen
des brevets

**INCOMPLETE SEARCH
SHEET C**

**Application Number**

EP 14 15 5254

Claim(s) completely searchable:
          1-11

Claim(s) searched incompletely:
          13-15

Claim(s) not searched:
          12

Reason for the limitation of the search:

The search has been restricted to the subject-matter indicated by the
applicant in his letter of 21.07.2014 filed in reply to the invitation
pursuant to Rule 62a(1).

**ANNEX TO THE EUROPEAN SEARCH REPORT
ON EUROPEAN PATENT APPLICATION NO.**                    EP 14 15 5254

This annex lists the patent family members relating to the patent documents cited in the above-mentioned European search report.
The members are as contained in the European Patent Office EDP file on
The European Patent Office is in no way liable for these particulars which are merely given for the purpose of information.

18-08-2014

| Patent document cited in search report | | Publication date | Patent family member(s) | | Publication date |
|---|---|---|---|---|---|
| WO 2013021815 | A1 | 14-02-2013 | CN | 103718029 A | 09-04-2014 |
| | | | EP | 2743690 A1 | 18-06-2014 |
| | | | JP | 2013036865 A | 21-02-2013 |
| | | | US | 2014158540 A1 | 12-06-2014 |
| | | | WO | 2013021815 A1 | 14-02-2013 |
| US 2014021047 | A1 | 23-01-2014 | KR | 20140012506 A | 03-02-2014 |
| | | | US | 2014021047 A1 | 23-01-2014 |
| WO 2012073009 | A2 | 07-06-2012 | NONE | | |
| WO 2011130312 | A1 | 20-10-2011 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CLARCKE et al.** *Nat. Nanotechnol.,* 2009, vol. 4, 265
- **BRANTON et al.** *Nat. Nanotechnol.,* 2008, vol. 26, 1146
- **VENTA et al.** *ACS Nano,* 2013, vol. 7, 4629-4636
- **GARAJ et al.** *Nature,* 2010, vol. 467 (7312), 190-193
- **TRAVERSI et al.** *Nat. Nanotechnol.,* 2013, vol. 8, 939-945
- **SCHNEIDER et al.** *Nat. Commun.,* 2013, vol. 4, 2619
- **FOLOGEA et al.** *Nano Lett,* 2005, vol. 5, 1734
- **NOVOSELOV et al.** *PNAS,* 2005, vol. 102, 10541-1053
- **LIU et al.** *Nano Lett.,* 2002, vol. 12, 1538-1544
- **CHIAPPE et al.** *J. Phys. Org. Chem,* 2005, vol. 18, 275-297
- **KESKIN et al.** *J. of Supercritical Fluids,* 2007, vol. 43, 150-180
- **CARDA-BROCH.** *Anal. Bioanal. Chem.,* 2003, vol. 375, 191-199
- **PETRONE et al.** *Nano Lett.,* 2012, vol. 12, 2751-2756
- **NANOSELOV.** *Science,* 2004, vol. 306, 666-669
- **WANUNU et al.** *Nat. Nanotechnol.,* 2010, vol. 5, 807-814
- **KOWALCYZK et al.** *Nanotechnology,* 2011, vol. 22, 315101
- **RAILLON et al.** *Nanoscale,* 2012, vol. 4, 4916-4924
- **WANANU et al.** *Biophys. J.,* 2008, vol. 95, 4716
- **STORM et al.** *Nano Lett.,* 2005, vol. 5, 1193